# FASCICULE DE BREVET EUROPEEN

(11) **EP 0 502 139 B1**
(45) Date de publication et mention de la délivrance du brevet: **14.12.1994**
(21) Numéro de dépôt: 91915024.3
(22) Date de dépôt: 09.09.1991
(51) Int. Cl.: B65D 83/02, A61J 1/00

(54) **DISPOSITIF POUR CONDITIONNER ET DISTRIBUER DES OBJETS STERILES**
EINRICHTUNG ZUR AUFBEWAHRUNG UND ABGABE VON KEIMFREIEN ARTIKELN
DEVICE FOR PACKAGING AND DISPENSING STERILE OBJECTS

(30) Priorité: 10.09.1990 FR 9011300
(43) Date de publication de la demande: 09.09.1992
(73) Titulaire: GUIGNET, Jean-Daniel, CH-1603 Grandvaux (CH); MODAK, Sohan, CH-1005 Lausanne (CH)
(72) Inventeur: GUIGNET, Jean-Daniel, CH-1603 Grandvaux (CH); MODAK, Sohan, CH-1005 Lausanne (CH)
(74) Mandataire: Nithardt, Roland
(86) Numéro de dépôt international: CH9100194
(87) Numéro de publication internationale: WO9204255

(56) Documents cités:
- DE-A- 2 821 346
- DE-A- 2 935 070

## Description

La présente invention concerne un dispositif pour conditionner et distribuer des objets stériles, comportant un boîtier stérile, un support agencé pour loger lesdits objets, un sélecteur mobile agencé pour libérer l'accès à au moins un des objets, et des moyens pour assurer une fermeture étanche du boîtier, dans lequel le sélecteur mobile est rotatif autour d'un axe central passant par le centre du support et comporte au moins un trou disposé de telle manière qu'il puisse être amené en coïncidence avec au moins un des objets placés dans le support.

Les objets stériles nécessitant des conditions de stockage et de distribution particulières sont notamment les aiguilles de seringues et les embouts allongés et coniques utilisés pour le transfert de volumes déterminés de substance liquide, dans le cadre d'applications biologiques et/ou médicales.

Dans les études biotechnologiques et de diagnostic médical, on est fréquemment amené à transférer des volumes liquides d'une éprouvette à une autre dans des conditions stériles. Ce procédé impose l'utilisation d'objets stériles à usage unique. Ces objets sont adaptés à un appareil du type pipette automatique, qui comporte un piston mobile engendrant un appel d'air suffisant pour provoquer une aspiration d'un volume de liquide équivalent au volume d'air déplacé par le piston mobile. Il est évident que, pour préserver les conditions de stérilité, les objets doivent être manipulés avec certaines précautions. Actuellement, les embouts sont livrés par des entreprises spécialisées dans des sacs souples en matière synthétique ou dans des boîtes contenant des supports, ces embouts étant disposés de telle manière que leur extrémité la plus large soit directement accessible par le haut de la boîte.

Ce conditionnement présente comme inconvénient majeur le fait que l'ouverture de la boîte ou du sac pour permettre le prélèvement d'un embout met en contact l'ensemble de ces embouts avec l'air ambiant ou, le cas échéant avec les doigts de l'opérateur, ce qui met en question les conditions de stérilité de ces embouts. Si les conditions de stérilité ne sont plus assurées, ces embouts doivent être relavés et autoclavés avant chaque utilisation. Même si ces opérations sont effectuées après chaque prélèvement, les embouts restants se dégradent s'ils sont amenés à subir plusieurs cycles successifs d'autoclavage.

Il est également nécessaire de prévoir un conditionnement particulier répondant aux mêmes critères que ceux requis dans le domaine médical pour les puces électroniques et les circuits intégrés maintenant couramment utilisés dans la fabrication de matériels ou installations liés à l'informatique.

La publication DE-A-2 935 070 décrit un dispositif tel que défini dans le préambule de la revendication 1, où le sélecteur rotatif comporte une fente radiale qui passe par-dessus plusieurs rangées circulaires des objets logés dans le boîtier. La fermeture étanche du boîtier est assurée seulement par un couvercle rabattable. Par conséquent, lorsqu'on ouvre le couvercle pour accéder à l'un des objets, les autres objets apparaissant dans la fente du sélecteur sont exposés à un risque de contamination.

La présente invention se propose de pallier les inconvénients des dispositifs antérieurs en réalisant un dispositif susceptible de préserver les conditions stériles ou propres des objets conditionnés, même si plusieurs prélèvements ponctuels sont effectués à des intervalles de temps plus ou moins longs.

Ce but est atteint par le dispositif selon l'invention, caractérisé en ce que le support est pourvu de cavités dans lesquelles sont engagés lesdits objets stériles, et en ce que les moyens pour assurer une fermeture étanche du boîtier comportent au moins une plaque de fermeture rotative autour dudit axe et dans laquelle est ménagée au moins une ouverture pouvant être amenée en coïncidence avec le trou du sélecteur au-dessus d'une des cavités du support, pour libérer l'accès à l'objet qui s'y trouve.

Selon un mode de réalisation préféré, le boîtier est sensiblement cylindrique et le support est constitué par une plaque circulaire.

De préférence, les cavités du support sont disposées selon des cercles concentriques à l'axe central, le sélecteur comporte autant de trous que le support comporte de cercles concentriques et la plaque de fermeture comporte une fente radiale dont la longueur correspond au moins à la distance entre le cercle de plus petit diamètre et celui de plus grand diamètre.

Dans la forme de réalisation où le support est une plaque circulaire, il est avantageux que l'axe central soit creux et qu'il comporte au moins une ouverture ménagée à l'extrémité extérieure et agencée pour être raccordée à une pompe à vide, et au moins une ouverture ménagée à l'intérieur du boîtier. Cette ouverture ménagée à l'intérieur du boîtier peut être disposée sous la plaque de fermeture.

De préférence, le boîtier et le support sont faits d'une seule pièce moulée.

La présente invention sera mieux comprise en référence à la description d'exemples de réalisation et du dessin annexé, dans lequel:
la figure 1 représente une vue en perspective d'une forme de réalisation avantageuse du dispositif selon l'invention,
la figure 2 représente une vue éclatée du dispositif illustré par la figure 1,
la figure 2A représente une vue éclatée d'une forme simplifiée du dispositif illustré par la figure 1, et
la figure 2B représente une vue en coupe des éléments du dispositif de la figure 2A.

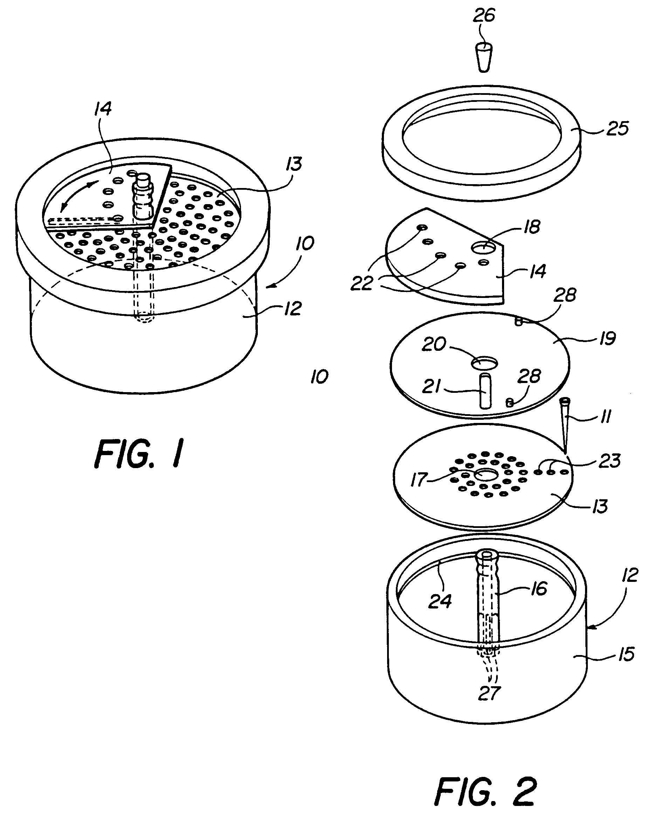

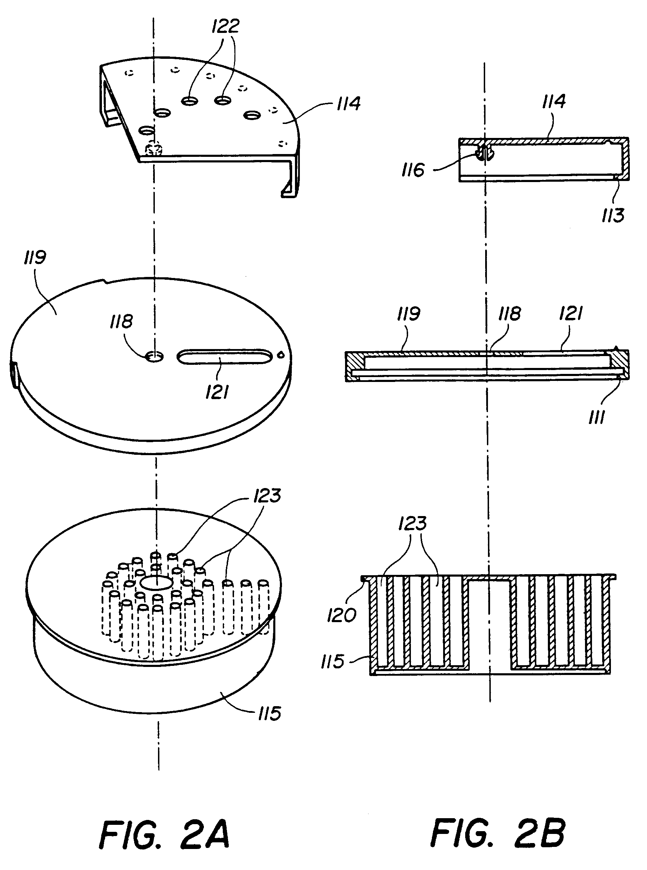

En référence aux figures 1 et 2, le dispositif 10 pour conditionner et distribuer des objets stériles allongés et coniques 11, se compose essentiellement d'un boîtier 12, contenant un support 13 agencé pour loger lesdits objets dans des rangées de trous, un sélecteur mobile 14 agencé pour libérer l'accès à au moins un objet, et des moyens pour assurer une fermeture étanche dudit boîtier. Lorsque les objets conditionnés sont des embouts coniques, ces embouts sont suspendus dans les trous du support 13 par leur extrémité la plus large.

Le boîtier a une forme sensiblement cylindrique et au centre de son fond 15 est fixé un axe 16 qui traverse une ouverture centrale 17 du support 13 ainsi qu'une ouverture centrale 18 du sélecteur 14. Entre le sélecteur 14 et le support 13 est montée une plaque de fermeture 19 comportant une ouverture centrale 20 dans laquelle est engagé l'axe 16, et une fente radiale 21 qui coopère avec des trous 22 ménagés dans le sélecteur 14. Il est à noter que les différents trous 22 sont décalés radialement par rapport à l'ouverture 18 de telle manière que chacun d'eux correspond à une des rangées de trous 23 du support 13. Ce support est en appui contre un épaulement 24 ménagé dans la paroi latérale du boîtier 12. Il est recouvert par une plaque de fermeture 19 et ces différents éléments sont maintenus en place par un élément annulaire 25.

L'axe 16 est creux et son extrémité supérieure est obturée par un bouchon 26. L'axe peut être raccordé, lorsque le bouchon de fermeture 26, est retiré à une pompe à vide qui permet de faire le vide à l'intérieur du boîtier. A cet effet, cet axe comporte à sa base un certain nombre de fentes ou trous axiaux 27 permettant de faire communiquer l'intérieur du boîtier avec le conduit ménagé à l'intérieur de l'axe 16.

Pour prélever un objet stérile contenu dans le boîtier 12, on fait tourner le sélecteur 14 de telle manière que l'un des trous 22 coïncide avec la fente 21 de la plaque de fermeture 19, celle-ci coïncidant avec des trous du support 13 et par conséquent avec les extrémités larges des objets 11, lorsque ces objets sont des embouts, logés dans ces trous. Deux points d'arrêt 28 sur le disque 19 permettent de limiter le déplacement latéral du sélecteur mobile 14. Dans le cadre de conditionnement d' embouts pour le transfert de volumes liquides, un appareil du type pipette automatique est adapté sur ledit embout. La pointe de cet embout est ensuite trempée dans le liquide à prélever et le piston de la pipette automatique se déplace pour aspirer un volume prédéterminé de ce liquide. Pour éviter que le liquide dans lequel l'embout est trempé soit contaminé, il est indispensable que cet embout soit stérile. Par ailleurs, cet embout doit également être stérile pour éviter de contaminer le volume de liquide prélevé destiné à être transféré pour une application biologique ou médicale. Dans le dispositif décrit précédemment, cette condition peut être réalisée grâce au fait que l'on peut sélectionner un seul embout sans risquer de contaminer les autres.

Le dispositif illustré par les figures 2A et 2B est une version simplifiée du dispositif décrit en référence aux figures 1 et 2 par le fait qu'il ne se compose que de trois éléments et ne peut pas être raccordé à une pompe à vide. Dans cette réalisation, le dispositif se compose d'un boîtier 115 servant aussi de support pour les objets 11 et comportant une série de cavités cylindriques 123 dont le diamètre et la profondeur peuvent être variables en fonction de la dimension de l'objet à conditionner, une plaque de fermeture 119 pourvue d'une fente radiale 121, et un sélecteur mobile 114 pourvu de trous 122 coopérant avec la fente radiale 121 de la plaque de fermeture 119 comme dans l'exemple précédent. Cette plaque de fermeture 119 comporte une ouverture centrale 118 coopérant avec un ergot 116 du sélecteur 114 permettant une interaction de ces deux éléments, ainsi qu'un rebord pourvu d'un décrochement 111 permettant la fixation de la plaque sur le boîtier 115 par encliquetage de ce décrochement sur le rebord proéminent 120 du boîtier. Le sélecteur mobile 114 est ensuite encliqueté sur l'ensemble formé par le boîtier et la plaque de fermeture, par l'intermédiaire de son rebord pourvu d'un décrochement 113. Le prélèvement des objets se fait de la même manière que dans le dispositif décrit plus haut. L'avantage de ce dispositif réside dans le fait que les trois éléments le composant peuvent être réalisés par moulage et ont donc un coût de fabrication peu élevé.

Dans le cadre de réalisations non représentées, le sélecteur mobile 14, la plaque de fermeture 19 et la plaque de support 13 du dispositif décrit en référence aux figures 1 et 2 peuvent être réalisés avec un rebord permettant leur fixation sur le boîtier par un encliquetage tel que celui décrit en référence au dispositif illustré par les figures 2A et 2B. On peut également réaliser des dispositifs dont le boîtier présente une section polygonale, par exemple carrée, triangulaire, hexagonale, etc.

## Revendications

1. Dispositif pour conditionner et distribuer des objets stériles, comportant un boîtier stérile (12, 115), un support (13, 115) agencé pour loger lesdits objets (11), un sélecteur mobile (14, 114) agencé pour libérer l'accès à au moins un des objets, et des moyens pour assurer une fermeture étanche du boîtier, dans lequel le sélecteur mobile (14, 114) est rotatif autour d'un axe central passant par le centre du support et comporte au moins un trou (22, 122) disposé de telle manière qu'il puisse être amené en coïncidence avec au moins un des objets (11) placés dans le support, caractérisé en ce que le support (13, 115) est pourvu de cavités (23, 123) dans lesquelles sont engagés lesdits objets stériles (11), et en ce que les moyens pour assurer une fermeture étanche du boîtier comportent au moins une plaque de fermeture (19, 119) rotative autour dudit axe et dans laquelle est ménagée au moins une ouverture (21, 121) pouvant être amenée en coïncidence avec le trou (22, 122) du sélecteur (14) au-dessus d'une des cavités (23, 123) du support, pour libérer l'accès à l'objet qui s'y trouve.

2. Dispositif selon la revendication 1, caractérisé en ce que boîtier (12) est sensiblement cylindrique et en ce que le support (13) est constitué par une plaque circulaire.

3. Dispositif selon la revendication 1 ou 2, caractérisé en ce que les cavités (23, 123) du support (13, 115) sont disposées selon des cercles concentriques à l'axe central, en ce que le sélecteur (14, 114) comporte autant de trous (22, 122) que le support comporte de cercles concentriques et en ce que la plaque de fermeture (19, 119) comporte une fente radiale (21, 121) dont la longueur correspond au moins à la distance entre le cercle de plus petit diamètre et celui de plus grand diamètre.

4. Dispositif selon la revendication 2, caractérisé en ce que l'axe central (16) est creux et comporte au moins une ouverture ménagée à l'extrémité extérieure et agencée pour être raccordée à une pompe à vide, et au moins une ouverture (27) ménagée à l'intérieur du boîtier.

5. Dispositif selon la revendication 4, caractérisé en ce que l'ouverture (27) ménagée à l'intérieur du boîtier est disposée sous la plaque de fermeture (19).

6. Dispositif selon la revendication 1, caractérisé en ce que le boîtier (115) et le support sont faits d'une seule pièce moulée.

## Claims

1. Device for packaging and distributing sterile objects comprising a sterile case (12, 115), a support (13, 115) designed to lodge said objects (11), a movable selector (14, 114)) designed to free the access to at least one object, and means to ensure an hermetic closure of the case, in which the movable selector (14, 114) rotates around a central axis passing through the centre of the support and comprises at least one hole (22, 122) disposed in such a manner that it can be brought to coincide with at least one of the objects (11) put in the support, characterized in that the support (13, 115) is provided with cavities (23, 123) in which said sterile objects (11) are inserted and in that the means to ensure an hermetic closure of the case comprises at least one closing plate (19, 119) moving around said axis and in which at least one opening (21, 121) which can be brought to coincide above one of the cavities (23, 123) of the support with the hole (22, 122) in the selector (14) is provided to free the access to the object housed in this cavity.

2. Device according to claim 1, characterized in that the case (12) is substantially cylindrical and in that the support (13) is constituted of a circular plate.

3. Device according to claims 1 or 2, characterized in that the cavities (23, 123) of the support (13, 115)) are disposed along circles concentric with the central axis, in that the selector (14, 114) comprises as many holes (22, 122) as the number of concentric circles of the support, and in that the closing plate (19,119) comprises a radial slit (21, 121) of a length corresponding to at least the distance between the circle of the smallest diameter and the circle of the largest diameter.

4. Device according to claim 2, characterized in that the central axis (16) is hollow and comprises at least one opening located at the external extremity and designed to be connected with a vacuum pump, and at least one opening (27) located inside the case.

5. Device according to claim 4, characterized in that the opening (27) inside the case is located below the closing plate (19).

6. Device according to claim 1, characterized in that the case (115) is integral with the support and made by moulding.

## Patentansprüche

1. Vorrichtung zur Aufbewahrung und Abgabe von keimfreien Artikeln, mit einem sterilen Gehäuse (12, 115), einer Aufbewahrungseinrichtung (13,115), die zur Aufnahme der Artikel (11) vorgesehen ist, einer beweglichen Trenneinrichtung (14,114), die dazu vorgesehen ist, den Zugang zu mindestens einem der Artikel freizugeben, und Mitteln zur Gewährleistung eines dichten Abschlusses des Gehäuses, wobei die bewegliche Trenneinrichtung (14,114) um eine Mittelachse, die durch die Mitte der Aufbewahrungseinrichtung führt, drehbar ist und wenigstens eine Öffnung (22,122) aufweist, die so angeordnet ist, daß wenigstens einer der Artikel, die sich in der Aufbewahrungseinrichtung befinden, durch die Öffnung austreten kann,
**dadurch gekennzeichnet,** daß die Aufbewahrungseinrichtung (13,115) mit Aussparungen (23,123) versehen ist, in die die sterilen Artikel (11) eingesetzt werden, und daß die Mittel zur Gewährleistung eines dichten Abschlusses des Gehäuses wenigstens eine Verschlußplatte (19,119) aufweisen, die um die Achse drehbar ist und in der wenigstens eine Öffnung (21,121) ausgespart ist, die zum Fluchten mit der Öffnung (22,122) in der Trenneinrichtung (14) über einer der Aussparungen (23,123) der Aufbewahrungseinrichtung gebracht werden kann, um den Zugang zu dem Artikel, der sich darin befindet, freizugeben.

2. Vorrichtung nach Anspruch 1,
**dadurch gekennzeichnet,** daß das Gehäuse (12) zylinderförmig ausgeführt ist, und daß die Aufbewahrungseinrichtung (13) aus einer kreisförmigen Platte gebildet ist.

3. Vorrichtung nach Anspruch 1 oder 2,
**dadurch gekennzeichnet,** daß die Aussparungen (23,123) der Aufbewahrungseinrichtung (13,115) in zur Mittelachse konzentrischen Kreisen angeordnet sind, daß die Anzahl der Öffnungen (22,122) der Trenneinrichtung (14,114) der Anzahl der konzentrischen Kreise der Aufbewahrungseinrichtung entspricht, und daß die Verschlußplatte (19,119) einen radialen Schlitz (21,121) aufweist, dessen Länge wenigstens dem Abstand zwischen dem Kreis mit dem kleinsten Durchmesser und dem mit dem größten Durchmesser entspricht.

4. Vorrichtung nach Anspruch 2,
**dadurch gekennzeichnet,** daß die Mittelachse (16) hohl und mit wenigstens einer Öffnung am äußersten Ende versehen und so angeordnet ist, daß sie mit einer Vakuumpumpe und wenigstens einer Öffnung (27), die im Inneren des Gehäuses ausgespart ist, verbunden werden kann.

5. Vorrichtung nach Anspruch 4,
**dadurch gekennzeichnet,** daß die Öffnung (27), die innen im Gehäuse ausgespart ist, unter der Verschlußplatte (19) angeordnet ist.

6. Vorrichtung nach Anspruch 1,
**dadurch gekennzeichnet,** daß das Gehäuse (115) und die Aufbewahrungseinrichtung als einstückiges Gußteil hergestellt sind.
